(19) Europäisches Patentamt

European Patent Office

Office européen des brevets

(11) Veröffentlichungsnummer: **0 521 981 B1**

(12) **EUROPÄISCHE PATENTSCHRIFT**

(45) Veröffentlichungstag der Patentschrift: **28.12.94**

(21) Anmeldenummer: **91906876.7**

(22) Anmeldetag: **21.03.91**

(86) Internationale Anmeldenummer:
**PCT/EP91/00556**

(87) Internationale Veröffentlichungsnummer:
**WO 91/15184 (17.10.91 91/24)**

(51) Int. Cl.⁵: **A61K 7/00**, A61K 7/08,
B01F 17/00

(54) **VERFAHREN ZUR HERSTELLUNG VON ÖL-IN-WASSER-CREMES.**

(30) Priorität: **30.03.90 DE 4010393**

(43) Veröffentlichungstag der Anmeldung:
**13.01.93 Patentblatt 93/02**

(45) Bekanntmachung des Hinweises auf die
Patenterteilung:
**28.12.94 Patentblatt 94/52**

(84) Benannte Vertragsstaaten:
**DE ES FR GB IT**

(56) Entgegenhaltungen:
**EP-A- 0 345 586**

**J. STEPHEN JELLINEK: "Formulation and
function of cosmetics", 1971, "Oil in water
emulsion", (London, GB) "Substances which
increase the viscosity of the aqueous phase", pages 146-152**

(73) Patentinhaber: **Henkel Kommanditgesellschaft
auf Aktien**

**D-40191 Düsseldorf (DE)**

(72) Erfinder: **SCHAMBIL, Fred
Niederstrasse 96
D-4019 Monheim (DE)**
Erfinder: **ZEIDLER, Ulrich
Heinrich-Lersch-Strasse 19
D-4000 Düsseldorf 13 (DE)**
Erfinder: **SHAMSAI, Soraya
Remigiusstrasse 20a
D-5000 Köln 41 (DE)**
Erfinder: **FÖRSTER, Thomas, Dr.
Adalbert-Stifter-Strasse 15
D-4006 Erkrath (DE)**
Erfinder: **TESMANN, Holger
Vennstrasse 61
D-4000 Düsseldorf 12 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

REMINGTON'S PHARMACEUTICAL SCIEN-CES;, 16 Edition, 1980, Philadelphia College of Pharmacy and Science, (US), "Emulsions", pages 1453-1454, see "Preparation", page 1454

**Beschreibung**

Die Erfindung betrifft ein Verfahren zur Herstellung von Cremes des Öl-in-Wasser-Emulsionstyps.

Es ist bekannt, daß Öl-in-Wasser-Emulsionen, die mit nichtionogenen Emulgatoren hergestellt sind, beim Erwärmen eine Phaseninversion erleiden, d. h., daß bei höheren Temperaturen die äußere, wäßrige Phase zur inneren Phase werden kann. Dieser Vorgang ist in der Regel reversibel, so daß sich beim Abkühlen wieder der ursprüngliche Emulsionstyp zurückbildet. F. Schambil, F. Jost und M. J. Schwuger berichten in "Progress in Colloid & Polymer Science 73, (1987), 37 - 47 über Eigenschaften kosmetischer Emulsionen von Paraffinöl und Paraffinöl-Esteröl-Gemischen und beschreiben dabei auch, daß Emulsionen, die oberhalb der Phaseninversionstemperatur hergestellt wurden, eine niedrigere Viskosität und eine hohe Lagerstabilität aufweisen.

Aus DE-A-38 19 193 sind flüssige Öl-in-Wasser-Emulsionen polarer Ölkomponenten bekannt, die bei einer Temperatur im Bereich des Phaseninversions-Temperaturintervalls oder darüber hergestellt wurden und daher niedrigviskos, feinteilig und sehr stabil sind. Bei Verwendung besonders ausgewählter Emulgatoren und Coemulgatoren sind solche Emulsionen so feinteilig, daß ihre Teilchen nicht mehr optisch sichtbar sind. Solche Emulsionen, die ein transparentes, bläulich opaleszierendes Aussehen haben, werden auch als Mikroemulsionen bezeichnet.

In der EP-A-345 586 geht es um die Herstellung niedrigviskoser und besonders stabiler Emulsionen.

Aus J. St. Jellinek "Formulation and function of cosmetics" 1971, S. 146-152, ist bekannt, daß Cetylalkohol als Coemulgator eine verdickende Wirkung auf O/W-Emulsionen hat.

Für kosmetische Zwecke ist eine hohe Feinteiligkeit und Stabilität zwar erwünscht, gleichzeitig sollten kosmetische Emulsionen aber für viele Zwecke nicht fließfähig sein, sondern eine salbenartige oder cremige Konsistenz aufweisen. Dies wurde bei der konventionellen Creme-Herstellung durch Erhöhung des Anteils der inneren Phase (Ölkomponente), durch Verwendung von festen Fetten oder Wachsen und durch Verwendung von Seifen oder anderen verdickenden oder gelbildenden anionischen Emulgatoren erreicht. Durch die genannten Maßnahmen wurden die Emulsionssysteme entweder erheblich verteuert oder die Feinteiligkeit und Stabilität verringert, die dermatologische Verträglichkeit oder das kosmetische Hautgefühl verschlechtert.

Es wurde nunmehr gefunden, daß sich Cremes vom Typ Öl-in-Wasser aus flüssigen Ölkomponenten mit großem Vorteil in der Weise herstellen lassen, daß man zunächst eine niedrigviskose, sehr feinteilige Öl-in-Wasser-Emulsion nach bekannten Verfahren unter Phaseninversion herstellt und diese nachträglich durch konsistenzgebende Coemulgatoren oder verdickende, hydrophile Polymere verdickt.

Gegenstand der Erfindung ist folglich ein Verfahren zur Herstellung von Öl-in-Wasser-Cremes aus flüssigen Ölkomponenten, indem man

(A) 1 Gewichtsteil der Ölkomponente mit

(B) 0,1 - 0,5 Gewichtsteilen eines nichtionischen Emulgators mit einem HLB-Wert von 11 - 15 und

(C) 0,1 - 0,2 Gewichtsteilen eines Coemulgators aus der Gruppe der gesättigten Fettalkohole mit 16 - 22 C-Atomen oder der Partialester von Polyolen mit 3 - 6 C-Atomen mit gesättigten Fettsäuren mit 14 - 22 C-Atomen und

(D) 1 - 6 Gewichtsteilen Wasser bei einer Temperatur oberhalb oder innerhalb des Phaseninversions-Temperaturbereiches in eine niedrigviskose, sehr feinteilige Öl-in-Wasser-Emulsion überführt, dadurch gekennzeichnet, daß man diese danach durch Einbringen eines lipophilen Konsistenzgebers vom Typ der unter (C) genannten Coemulgatoren oder eines wasserlöslichen Polymeren bis zur Cremekonsistenz verdickt, bei der die Emulsion bei 20°C ein plastisches Verhalten mit einer Fließgrenze von mindestens 5 Pascal, bevorzugt von 10 bis 50 Pascal aufweist.

Das Einbringen des lipophilen Konsistenzgebers kann sowohl oberhalb als auch innerhalb oder unterhalb des Phaseninversions-Temperaturbereiches erfolgen.

Die nach diesem erfindungsgemäßen Verfahren erhaltenen Öl-in-Wasser-Cremes zeichnen sich durch eine besonders hohe Feinteiligkeit und Stabilität bei gleichzeitig sehr guter kosmetisch erwünschter Cremekonsistenz und Verteilbarkeit aus.

Als Cremekonsistenz soll dabei ein rheologischer Zustand bezeichnet werden, bei welchem die Emulsion bei Raumtemperatur von 20 °C ein plastisches Verhalten mit einer Fließgrenze von mindestens 5 Pascal [Pa], bevorzugt von 10 bis 50 Pascal zeigt.

Als Ölkomponenten können an sich alle bei Raumtemperatur (20 °C) flüssigen, wasserunlöslichen, verzweigten oder linearen, physiologisch verträglichen Kohlenwasserstoffe, Ether oder Ester sowie fette Öle (Triglyceride) eingesetzt werden. Es können aber auch feste oder höherschmelzende Paraffine, Ester, Wachse oder Fette in solchen Mengen mitverwendet werden, daß die Mischung mit den flüssigen Ölkomponenten bei 20 °C flüssig bleibt.

Bevorzugt geeignet sind als Ölkomponenten Paraffinöle und synthetisch hergestellte Kohlenwasserstoffe, z. B. flüssige Polyolefine oder definierte Kohlenwasserstoffe, z. B. Alkylcyclohexane wie z. B. das 1,3-Diisooctylcyclohexan.

Gut geeignet sind aber auch Mono- und Diester der Formeln

(I)   $R^1$-COOR$^2$

(II)   $R^2$-OOC-$R^3$-COOR$^2$

(III)   $R^1$-COO-$R^3$-OOC-$R^1$,

worin $R^1$ und $R^2$ Alkylgruppen mit 1 - 22 C-Atomen oder Alkenylgruppen mit 8 - 22 C-Atomen und $R^3$ Alkylengruppen mit 2 - 16 C-Atomen sind, und die mindestens 10 C-Atome enthalten, und/oder Fettsäuretriglyceride von Fettsäuren mit 8 - 22 C-Atomen.

Ölkomponenten vom Typ der Mono- und Diester der Formeln I, II und III sind als kosmetische und pharmazeutische Ölkomponenten sowie als Gleit- und Schmiermittelkomponenten bekannt. Unter den Mono- und Diestern dieser Art kommt den bei Raumtemperatur (20 °C) flüssigen Produkten die größte Bedeutung zu. Als Ölkomponenten geeignete Monoester (I) sind z. B. die Methylester und Isopropylester von Fettsäuren mit 12 - 22 C-Atomen, wie z. B. Methyllaurat, Methylstearat, Methyloleat, Methylerucat, Isopropylpalmitat, Isopropylmyristat, Isopropylstearat, Isopropyloleat. Andere geeignete Monoester sind z. B. n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-stearat, 2-Octyldodecyl-palmitat, Oleyloleat, Oleylerucat, Erucyloleat sowie Ester, die aus technischen aliphatischen Alkoholgemischen und technischen aliphatischen Carbonsäuren erhältlich sind, z. B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z. B. im Jojobaöl oder im Spermöl vorliegen.

Geeignete Dicarbonsäuren (II) sind z. B. Di-n-butyl-adipat, Di-n-butyl-sebacat, Di-(2-ethylhexyl)-adipat, Di-(2-hexyldecyl)-succinat und Di-isotridecyl-acelaat. Geeignete Diolester (III) sind z. B. Ethylenglycol-dioleat, Ethylenglycol-di-isotridecanoat, Propylenglycol-di-(2-ethylhexanoat), Butandiol-di-isostearat und Neopentylglycol-di-caprylat.

Als Fettsärnretriglyceride können natürliche, pflanzliche Öle, z. B. Olivenöl, Sonnenblumenöl, Sojaöl, Erdnußöl, Rapsöl, Mandelöl, Palmöl, aber auch die flüssigen Anteile des Kokosöls oder des Palmkernöls sowie tierische Öle, wie z. b. Klauenöl, die flüssigen Anteile des Rindertalges oder auch synthetische Triglyceride, wie sie durch Veresterung von Glycerin mit Fettsäuren mit 8 - 22 C-Atomen erhalten werden, z. B. Triglyceride von Caprylsäure-CaprinsäureGemischen, Triglyceride aus technischer Ölsäure oder aus Palmitinsäure-Gemischen.

Als Emulgatoren (B) geeignete nichtionische Emulgatoren sind gekennzeichnet durch eine lipophile, bevorzugt lineare Alkyl- oder Acylgruppe und eine hydrophile, aus niedermolekularen Glycol-, Glucose- und Polyolethern gebildete Gruppe.

Als Emulgatoren (B) geeignete nichtionogene Ethylenoxidanlagerungsprodukte an Fettalkohole mit 16 - 22 C-Atomen sind handelsüblich. Die technischen Produkte stellen Gemische homologer Polyglycolether der Ausgangsfettalkohole dar, deren mittlerer Oxethylierungsgrad der angelagerten Molmenge an Ethylenoxid entspricht. Als Emulgatoren können auch Ethylenoxidanlagerungsprodukte an Partialester aus einem Polyol mit 3 - 6 C-Atomen und Fettsäuren mit 14 - 22 C-Atomen verwendet werden. Solche Produkte werden z. B. durch Ethoxylierung von Glycerinmonostearat, Glycerinmonopalmitat, oder von Mono- und Di-Fettsäureestern des Sorbitans, z. B. von Sorbitanmonostearat oder Sorbitansesquioleat hergestellt. Die für das erfindungsgemäße Verfahren geeigneten Emulgatoren sollen einen HLB-Wert von 11 - 15 aufweisen. Unter dem HLB-Wert (Hydrophil-Lipophil-Balance) soll ein Wert verstanden werden, der errechnet werden kann gemäß

$$HLB = \frac{100 - L}{5}$$

4

worin L der Gewichtsanteil der lipophilen Gruppen, d. h. der Fettalkyl- oder Fettacylgruppen in Gewichtsprozent in den Ethylenoxidanlagerungsprodukten ist.

Zusätzlich zum Emulgator ist zur Herstellung der Öl-in-Wasser-Emulsionen nach dem erfindungsgemäßen Verfahren ein Coemulgator (C) erforderlich. Der Coemulgator ist aufgrund seiner Hydrophilie selbst nicht zur Herstellung von Öl-in-Wasser-Emulsionen geeignet, gemeinsam mit den oben definierten Emulgatoren lassen sich jedoch besonders stabile und feinteilige Emulsionen der polaren Ölkomponenten herstellen. Als Coemulgatoren sind erfindungsgemäß solche vom Typ der gesättigten Fettalkohole mit 16 - 22 C-Atomen, z. B. Cetylalkohol, Stearylalkohol, Arachidylalkohol oder Behenylalkohol oder Gemische dieser Alkohole geeignet, wie sie bei der technischen Hydrierung von pflanzlichen und tierischen Fettsäuren mit 16 - 22 C-Atomen oder der entsprechenden Fettsäuremethylester erhalten werden. Weiterhin eignen sich als Coemulgatoren Partialester aus einem Polyol mit 3 - 6 C-Atomen und gesättigten und/oder ungesättigten Fettsäuren mit 14 - 22 C-Atomen. Solche Partialester sind z. B. die Monoglyceride von Palmitin-, Stearinsäure und Öl-Säure, die Sorbitanmono- und/oder -diester von Myristinsäure, Palmitinsäure, Stearinsäure oder von Mischungen dieser Fettsäuren, die Monoester aus Trimethylolpropan, Erythrit oder Pentaerythrit und gesättigten Fettsäuren mit 14 - 22 C-Atomen. Als Monoester werden auch die technischen Monoester verstanden, die durch Veresterung von 1 Mol Polyol mit 1 Mol Fettsäure erhalten werden und die ein Gemisch aus Monoester, Diester, Triester und ggf. unverestertem Polyol darstellen.

Besonders bevorzugt ist es, wenn sich bei dem erfindungsgemäßen Verfahren zunächst eine Emulsion bildet, die so feinteilig ist, daß die Emulsionströpfchen submikroskopisch fein sind und die Emulsion ein transparentes, bläulich opaleszierendes Aussehen hat. Solche Emulsionen werden auch Mikroemulsionen genannt. Mikroemulsionen werden erhalten, wenn Art und Mengenverhältnisse von Ölkomponenten Emulgatoren und Coemulgatoren in der bevorzugten Weise aufeinander abgestimmt sind. So ist es z. B. bevorzugt, daß die Ölkomponente (A) aus 50 - 100 Gew. -% eines Kohlenwasserstofföls und 0 - 25 Gew.-% eines Mono- oder Diesters der Formeln I, II oder III und 0 - 25 % eines Fettsäuretriglycerids von Fettsäuren mit 8 - 22 C-Atomen besteht.

Als nichtionische Emulgatoren (B) werden bevorzugt Anlagerungsprodukte von 8 - 16 Mol Ethylenoxid an gesättigte Fettalkohole mit 16 - 22 C-Atomen verwendet. Wenn eine polare Ölkomponente, z. B. ein Ester der Formeln I, II oder III oder ein Triglycerid eingesetzt wird, ist es besonders bevorzugt, als Emulgator ein Anlagerungsprodukt von 8 - 15 Mol Ethylenoxid an einen gesättigten, linearen Fettalkohol mit 20 - 22 C-Atomen einzusetzen.

Das erfindungsgemäße Verfahren kann in der Weise durchgeführt werden, daß zunächst die Phaseninversionstemperatur bestimmt wird, indem man eine Probe der auf übliche Weise hergestellten Emulsion unter Verwendung eines Leitfähigkeitsmeßgerätes erhitzt und die Temperatur bestimmt, bei der die Leitfähigkeit stark abnimmt. Die spezifische Leitfähigkeit der zunächst vorhandenen Öl-in-Wasser-Emulsion nimmt üblicherweise über einen Temperaturbereich von 2 - 8 °C von anfänglich über 1 Millisiemens pro cm (mS/cm) auf Werte unter 0,1 mS/cm ab. Dieser Temperaturbereich wird hier als Phaseninversions-Temperaturbereich bezeichnet.

Nachdem der Phaseninversions-Temperaturbereich bekannt ist, kann man das erfindungsgemäße Verfahren entweder in der Weise durchführen, daß man die zunächst wie üblich hergestellte Emulsion nachträglich auf eine Temperatur erhitzt, die innerhalb oder oberhalb des Phaseninversions-Temperaturbereichs liegt, oder in der Weise, daß man bereits bei der Herstellung der Emulsion eine Temperatur wählt, die innerhalb oder oberhalb des Phaseninversions-Temperaturbereichs liegt.

Die nachträgliche Verdickung wird bevorzugt in der Weise durchgeführt, daß 0,01 - 0,3 Gewichtsteile (pro 1 Gewichtsteil der Ölkomponente) eines lipophilen Konsistenzgebers in die flüssige, feinteilige Emulsion oder Mikroemulsion einemulgiert wird. Hierzu wird der Konsistenzgeber über seinen Schmelzpunkt erwärmt und intensiv mit der auf gleiche Temperatur, die auch im Phaseninversions-Temperaturbereich liegen kann, erwärmten Emulsion vermischt. Nach dem Abkühlen auf Raumtemperatur (20 °C) bildet sich dann eine nicht mehr fließfähige Creme.

Als lipophile Konsistenzgeber eignen sich Produkte des gleichen Typs, wie sie vorher als Coemulgatoren (C) beschrieben wurden. Bevorzugt geeignet sind lineare, gesättigte Fettalkohole mit 16 - 22 C-Atomen, insbesondere Cetylalkohol und Stearylalkohol sowie Glycerinmonostearat und Gemische aus Glycerinmono- und -distearat.

Die nachträgliche Verdickung durch Einbringen eines wasserlöslichen Polymeren geschieht bevorzugt in der Weise, daß man eine wäßrige Lösung von 0,005 bis 0,1 Gewichtsteilen eines wasserlöslichen Polymeren (pro 1 Gewichtsteil der Ölkomponente) der flüssigen, feinteiligen Emulsion oder Mikroemulsion zusetzt. Als wasserlösliche, verdickende Polymere eignen sich alle wasserlöslichen Polymeren mit einem Molekulargewicht von 500 000 bis 5 000 000. Geeignete Polymere können natürliche Stoffe, wie z. B. Pflanzengummen, Guar, lösliche Stärke oder Biopolymere, wie z. B. Xanthan-Gum oder auch wasserlösliche Derivate solcher Naturprodukte, wie z. B. Carboxymethylstärke, Hydroxyethylcellulose, Hydroxypropylguar u. a. sein.

Weiterhin eignen sich wasserlösliche synthetische Polymere wie z. B. Polyvinylpyrrolidon, Polyvinylalkohol, Polyethylenoxide, Polyacrylamide sowie Polymerisate und Copolymerisate der Acrylsäure und Methacrylsäure.

Als besonders geeignet haben sich vernetzte Acrylsäure-Polymerisate oder -Copolymerisate mit einem mittleren Molekulargewicht von 500 000 bis 5 000 000 in Form der wasserlöslichen Salze sowie wasserlösliche, nichtionische Celluloseether oder Gemische davon erwiesen.

Als wasserlösliche nichtionische Celluloseether eignen sich z. B. Methylcellulose, Hydroxyethylcellulose, Hydroxypropylcellulose und/oder Methylhydroxypropylcellulosen. Bevorzugt geeignet sind Hydroxypropylcellulose und Methyl-Hydroxypropylcellulose. Hydroxypropylcellulosen sind im Handel z. B. unter dem Warenzeichen KLUCEL[(R)] (der Fa. Hercules), Methylhydroxypropylcellulosen unter dem Warenzeichen BENECEL[(R)]MP (der Fa. Aqualon GmbH & Co. KG) erhältlich.

Die verschiedenen handelsüblichen Typen solcher nichtionogener Celluloseether unterscheiden sich durch den Substitutionsgrad und den Abbaugrad der Cellulose (d. h. das mittlere Molekulargewicht), wobei sich Qualitäten mit unterschiedlicher Lösungsviskosität ergeben.

Geeignete Methyl-hydroxypropylcellulosen haben in 2 Gew.-%iger wäßriger Lösung bei 20 °C eine Viskosität von 40 bis 40 000 mPas (gemessen mit einem Brookfield-Rotationsviskosimeter bei 20 UpM).

Als vernetzte Acrylsäure-Polymerisate eignen sich Produkte, die durch Copolymerisation von Acrylsäure mit 0,1 - 4,0 Gew.-% eines Polyalkenyl-polyethers eines mehrwertigen Alkohols mit mehr als einer Alkenylethergruppe im Molekül als Vernetzungsmittel erhalten werden. Ein Beispiel für ein solches Vernetzungsmittel ist z. B. Polyallylsucrose.

Gegebenenfalls können bei der Herstellung der vernetzten Acrylsäure-Polymerisate auch weitere Comonomere in Mengen bis zu 59 Gew.-% der Monomerenmischung eingesetzt werden. Geeignete Comonomere sind z. B. Maleinsäureanhydrid, N-Methylacrylamid, Methyl-vinylether oder Mischungen solcher zusätzlicher Monomerer. Solche vernetzte Acrylsäurepolymerisate sind z. B. aus US-PS-2 798 053 bekannt und unter dem Warenzeichen CARBOPOL[(R)] (der Fa. Goodrich) im Handel. Die vernetzten Acrylsäure-Polymerisate lassen sich in Wasser dispergieren, die starke Verdickungswirkung wird jedoch erst erreicht, wenn die Polymerisate durch anorganische Basen wie z. B. Natriumhydroxid, Kaliumhydroxid, Ammoniak oder durch niedermolekulare Amine oder Alkanolamine in die Salzform überführt werden.

Geeignete Acrylsäure-Polymerisate haben in 1 Gew.-%iger wäßriger Lösung, neutralisiert mit Natronlauge bei einem pH-Wert von 7 - 8 bei 20 °C eine Viskosität von 1 000 - 100 000 mPas (gemessen mit einem Brookfield-Rotationsviskosimeter bei 20 UpM).

Das Zusetzen der wäßrigen Lösung oder Quellung der Polymeren und das Vermischen mit der zuvor hergestellten feinteiligen, flüssigen Emulsion oder Mikroemulsion geschieht am besten in der Weise, daß man sowohl die Polymerlösung als auch die Emulsion auf eine Mischtemperatur von 30 - 80 °C bringt und dann unter intensivem Rühren, gegebenenfalls unter Verwendung statischer oder dynamischer Misch- oder Emulgiervorrichtungen, mischt und homogenisiert. Die Mischtemperatur wird dabei so hoch gewählt, daß die dabei entstehende verdickte Emulsion noch im fließfähigen Zustand bleibt. Nach dem Abkühlen auf Raumtemperatur (20 °C) erstarrt die verdickte Emulsion dann zur nicht mehr fließfähigen, plastischen Creme.

Eine besonders gute Verdickungswirkung wird auch dadurch erzielt, daß man sowohl einen lipophilen Konsistenzgeber als auch ein wasserlösliches Polymerisat zur Verdickung verwendet.

Die folgenden Beispiele sollen den Erfindungsgegenstand näher erläutern ohne ihn hierauf zu beschränken:

**Beispiele**

1. Herstellung der Emulsionen (übliche Arbeitsweise)

Die Ölkomponenten, Emulgatoren und Coemulgatoren wurden gemischt und auf eine Temperatur oberhalb des Schmelzpunktes der Mischung erwärmt und homogenisiert. Dann wurde die Schmelze unter Rühren in das Wasser, welches auf etwa die gleiche Temperatur erhitzt war, einemulgiert. Die Zusammensetzung der Emulsionen ist der Tabelle I zu entnehmen.

2. Ermittlung der Phaseninversionstemperatur

Unter Verwendung einer Leitfähigkeitsmeßbrücke (Fa. Radiometer, Kopenhagen) wurde die elektrische Leitfähigkeit der Emulsionen in Abhängigkeit von der Temperatur ermittelt. Zu diesem Zweck wurde die Emulsion zunächst auf + 20 °C abgekühlt. Bei dieser Temperatur zeigten die Emulsionen eine Leitfähigkeit von über 1 Millisiemens pro cm (mS/cm), d. h. sie lagen als Öl-in-Wasser-Emulsionen vor.

Durch langsames Erwärmen mit einer Heizrate von ca. 0,5 °C/min, die mit Hilfe eines Temperatur-Programmgebers in Verbindung mit einem Kryostaten gesteuert wurde, wurde ein Leitfähigkeitsdiagramm erstellt. Der Temperaturbereich, innerhalb welchem die Leitfähigkeit auf Wert unterhalb 0,1 mS/cm abfiel, wurde als Phaseninversions-Temperaturbereich notiert. Bei allen in Tabelle I aufgeführten Emulsionen lag dieser Temperaturbereich unter 100 °C (Tabelle I, Phaseninversion).

3. Erfindungsgemäße Herstellung der Emulsionen

Die Emulsionen wurden, wie unter 1. beschrieben, hergestellt und dann kurzzeitig (ca. 1 Minute) auf eine Temperatur im Phaseninversions-Temperaturbereich oder wenig darüber erhitzt (in allen Beispielen 95 °C). Dann wurden die Emulsionen rasch, d. h. mit einer Abkühlrate von ca. 2 °C pro Minute, unter Rühren auf Raumtemperatur abgekühlt. Nach 24 Stunden wurde die Fließfähigkeit und die Fließgrenze bestimmt. Alle gemäß Tabelle I, Beispiel 1.1 - 1.8 hergestellten Mikroemulsionen waren flüssig, d. h. beweglich unter der Einwirkung der Schwerkraft. Die Fließgrenze lag weit unter 0,1 Pa (20 °C).

4. Erfindungsgemäße Herstellung der Cremes

Die Cremes 2.1 - 2.8 wurden aus den Mikroemulsionen 1.1 - 1.8 durch Zugabe der in Tabelle I angegebenen Konsistenzgeber in den angegebenen Mengen bei der angegebenen Mischtemperatur hergestellt.

In Beispiel 2.1 - 2.6 wurde dazu der Konsistenzgeber (C) auf Mischtemperatur erwärmt. Unter intensivem Rühren wurde die auf die gleiche Temperatur erwärmte Mikroemulsion zugegeben. In Beispiel 2.7 und 2.8 wurde die auf Mischtemperatur erwärmte Polymerlösung mit der auf gleiche Temperatur erwärmten Mikroemulsion vermischt. Nach dem Abkühlen auf 20 °C bildeten sich Cremes, die unter dem Einfluß der Schwerkraft keine Fließfähigkeit zeigten.

Nach 24 Stunden Lagerzeit bei 20 °C wurde die Fließgrenze in [Pa] in einem Rotationsviskosimeter (Carrimed controlled Stress Rheometer) bestimmt.

# Tabelle I:

| Mikroemulsion Nr. | V | 1.1 | 1.2 | 1.3 | 1.4 | 1.5 | 1.6 | 1.7 | 1.8 | 1.9 | 1.10 | 1.11 |
|---|---|---|---|---|---|---|---|---|---|---|---|---|
| Paraffinöl (DAB6), dickfl.(Gew.-%) | 20 | 20 | 20 | 20 | 20 | 20 | 15 | 20 | 20 | 20 | 20 | 20 |
| Decyloleat (Gew.-%) | | | | | | | 5 | | | | | |
| Cetyl-/Stearylalk.[c] + 12 EO (Gew.-%) | 4 | 4 | 4 | 4 | 4 | 4 | 4 | 4 | 4 | 4 | 4 | 4 |
| Cetyl-/Stearylalk.[c] (Gew.-%) | 3 | 3 | 3 | 3 | 3 | 3 | - | 3 | | | 3 | 3 |
| Glycerinmono-/distearat (Gew.-%) | 2[a] | | | | | | 3[a] | | 3[b] | 3[b] | - | - |
| Wasser (Gew.-%) | 71 | 73 | 73 | 73 | 73 | 73 | 73 | 73 | 73 | 73 | 73 | 73 |
| Phaseninversion [°C] | - | 81-91 | 81-91 | 81-91 | 81-91 | 81-91 | 69-77 | 81-89 | 65-74 | 65-74 | 81-89 | 81-89 |
| Fließgrenze 20 °C [Pa] | 1,3 | unter 0,1 [Pa], dünnflüssige Mikroemulsion | | | | | | | | | | |

EP 0 521 981 B1

Tabelle II:

| Verdickung: Creme Nr. | | | 2.1 | 2.2 | 2.3 | 2.4 | 2.5 | 2.6 | 2.7 | 2.8 | 2.9 | 2.10 | 2.11 |
|---|---|---|---|---|---|---|---|---|---|---|---|---|---|
| Mikroemulsion | Nr. | V | 1.1 | 1.2 | 1.3 | 1.4 | 1.5 | 1.6 | 1.7 | 1.8 | 1.9 | 1.10 | 1.11 |
| | Gew.-% | | 98 | 98 | 95,5 | 96 | 96 | 96 | 98 | 90 | 90 | 85 | 90 |
| Cetyl-/Stearylalk.[c] (Gew.-%) | | | | 2 | - | 4 | 4 | 4 | | | | | |
| Glycerinmono-/distearat (Gew.-%) | | | 2[a] | | 2[a] | | | | 2[a] | 10[b] | 10[b] | | |
| Carbopol[R] 940 Lsg. (2 % in $H_2O$/KOH) | | | | | 2,5 | | | | | | | 15 | 10 |
| Mischtemperatur (°C) | | 75 | 75 | 70 | 70 | 65 | 95 | 70 | 75 | 65 | 80 | 60 | 95 |
| Fließgrenze 20 °C [Pa] | | 1,3 | 11 | 14 | 22 | 27 | >15 | 16 | 11 | 14 | 36 | 43 | 9 |

EP 0 521 981 B1

EP 0 521 981 B1

Erläuterungen zur Tabelle I:

a) Die mit dem Index a) gekennzeichneten Mengen Glycerinmono-/di-stearat bestanden aus einem technischen Partialglycerid aus einem Palmitin-Stearinsäuregemisch (30 : 70) mit einem Anteil von 50 Gew.-% Monoglycerid, 40 Gew.-% Diglycerid und 10 Gew.-% Triglycerid.

b) Die mit dem Index b) gekennzeichneten Mengen Glycerinmono-/di-stearat bestanden aus einem technischen Partialglycerid aus einem Palmitin-Stearinsäuregemisch (50 : 50) mit einem Anteil von 42 Gew.-% Monoglycerid, 38 Gew.-% Diglycerid und 20 Gew.-% Triglycerid.

c) Das mit dem Index c) gekennzeichnete Cetyl-Stearylalkohol-Gemisch bestand aus ca. 50 Gew.-% Cetylalkohol und 50 Gew.-% Stearylalkohol.

**Patentansprüche**

1. Verfahren zur Herstellung von Öl-in-Wasser-Cremes aus flüssigen Ölkomponenten, indem man
   (A) 1 Gewichtsteil der Ölkomponenten mit
   (B) 0,1 - 0,5 Gewichtsteilen eines nichtionischen Emulgators mit einem HLB-Wert von 11 - 15 und
   (C) 0,1 - 0,2 Gewichtsteilen eines Coemulgators aus der Gruppe der gesättigten Fettalkohole mit 16 - 22 C-Atomen oder der Partialester von Polyolen mit 3 - 6 C-Atomen mit gesättigten Fettsäuren mit 14 - 22 C-Atomen und
   (D) 1 - 6 Gewichtsteilen Wasser bei einer Temperatur oberhalb oder innerhalb des Phaseninversions-Temperaturbereiches in eine niedrigviskose, sehr feinteilige Öl-in-Wasser-Emulsion überführt, dadurch gekennzeichnet, daß man diese danach durch Einbringen eines lipophilen Konsistenzgebers vom Typ der unter (C) genannten Coemulgatoren und/oder eines wasserlöslichen Polymeren bis zur Cremekonsistenz verdickt, bei der die Emulsion bei 20°C ein plastisches Verhalten mit einer Fließgrenze von mindestens 5 Pascal, bevorzugt von 10 bis 50 Pascal aufweist.

2. Verfahren nach Anspruch 1, dadurch gekennzeichnet, daß die Verdickung durch Einemulgieren von 0,01 - 0,3 Gewichtsteilen eines lipophilen Konsistenzgebers oder durch Zugabe einer wässrigen Lösung von 0,005 - 0,1 Gewichtsteilen eines wasserlöslichen Polymeren mit einem mittleren Molekulargewicht von 500 000 - 5 000 000 Dalton erfolgt.

3. Verfahren nach Anspruch 1 oder 2, dadurch gekennzeichnet, daß als nichtionischer Emulgator (B) ein Anlagerungsprodukt von 8 - 16 Mol Ethylenoxid an einem gesättigten Fettalkohol mit 16 - 22 C-Atomen verwendet wird.

4. Verfahren nach einem der Ansprüche 1 - 3, dadurch gekennzeichnet, daß die Ölkomponente (A) aus 50 - 100 Gew.-% eines Kohlenwasserstofföls und 0 - 25 Gew.-% eines Mono- und/oder Diesters der Formeln

$R^1$-COO-$R^2$
$R^2$-OOC-$R^3$-COO-$R^2$

10

und

R$^1$-COO-R$^3$-OOC-R$^1$ ,

worin R$^1$ und R$^2$ Alkylgruppen mit 1 - 22 C-Atomen oder Alkenylgruppen mit 8 - 22 C-Atomen und R$^3$ Alkylengruppen mit 2 - 16 C-Atomen sind und die mindestens 10 C-Atome enthalten, und 0 - 25 Gew.-% eines Fettsäuretriglycerids von Fettsäuren mit 8 - 22 C-Atomen besteht.

5.  Verfahren nach einem der Ansprüche 1 - 4, dadurch gekennzeichnet, daß der lipophile Konsistenzgeber ausgewählt ist aus der Gruppe Cetylalkohol, Stearylalkohol, Glycerinmonostearat und Gemischen aus Glycerinmono- und Distearat.

6.  Verfahren nach einem der Ansprüche 1 - 5, dadurch gekennzeichnet, daß das wasserlösliche Polymere ausgewahlt ist aus vernetzten Acrylsäure-Polymerisaten oder Copolymerisaten mit einem mittleren Molekulargewicht von 500 000 bis 5 000 000 in Form der wasserlöslichen Salze und wasserlöslichen, nichtionischen Celluloseethern oder Gemischen davon.

## Claims

1.  A process for the production of oil-in-water creams from liquid oil components by conversion of
    (A) 1 part by weight of the oil components with
    (B) 0.1 to 0.5 part by weight of a nonionic emulsifier having an HLB value of 11 to 15 and
    (C) 0.1 to 0.2 part by weight of a co-emulsifier from the group of saturated fatty alcohols containing 16 to 22 carbon atoms or partial esters of polyols containing 3 to 6 carbon atoms with saturated fatty acids containing 14 to 22 carbon atoms and
    (D) 1 to 6 parts by weight water
    into a low-viscosity, very fine-particle oil-in-water emulsion at a temperature above or within the phase inversion temperature range, characterized in that the emulsion thus formed is subsequently thickened to the consistency of a cream, at which the emulsion exhibits plastic behaviour with a yield point of at least 5 pascal and preferably 10 to 50 pascal, by introduction of a lipophilic consistency generator selected from the co-emulsifiers mentioned in (C) and/or of a water-soluble polymer.

2.  A process as claimed in claim 1, characterized in that thickening is carried out by incorporating 0.01 to 0.3 part by weight of a lipophilic consistency generator in the emulsion or by addition of an aqueous solution of 0.005 to 0.1 part by weight of a water-soluble polymer having an average molecular weight in the range from 500,000 to 5,000,000 dalton.

3.  A process as claimed in claim 1 or 2, characterized in that an adduct of 8 to 16 moles of ethylene oxide with a saturated C$_{16-22}$ fatty alcohol is used as the nonionic emulsifier (B).

4.  A process as claimed in any of claims 1 to 3, characterized in that the oil component (A) consists of 50 to 100% by weight of a hydrocarbon oil and
    0 to 25% by weight of a monoester and/diester corresponding to the following formulae:

    (I)     R$^1$COOR$^2$

    (II)    R$^2$-OOC-R$^3$-COOR$^2$

    (III)   R$^1$-COO-R$^3$-OOC-R$^1$

    in which R$^1$ and R$^2$ are C$_{1-22}$ alkyl groups or C$_{8-22}$ alkenyl groups and R$^3$ represents C$_{2-16}$ alkylene groups and which contain at least 10 carbon atoms, and
    0 to 25% by weight of a fatty acid triglyceride of C$_{8-22}$ fatty acids.

5.  A process as claimed in any of claims 1 to 4, characterized in that the lipophilic consistency generator is selected from the group consisting of cetyl alcohol, stearyl alcohol, glycerol monostearate and mixtures of glycerol mono- and distearate.

6. A process as claimed in any of claims 1 to 5, characterized in that the water-soluble polymer is selected from crosslinked acrylic acid polymers or copolymers having an average molecular weight in the range from 500,000 to 5,000,000 in the form of the water-soluble salts and water-soluble, nonionic cellulose ethers or mixtures thereof.

**Revendications**

1. Procédé de préparation de crèmes d'huile dans l'eau à partir de composants d'huile liquides, dans lequel on transforme :

    A) 1 partie en poids des composants de l'huile avec

    B) 0,1 - 0,5 partie en poids d'un émulsionnant non ionique avec une valeur HLB de 11-15 et

    C) 0,1-0,2 parties en poids d'un co-émulsionnant du groupe des alcools gras saturés avec 16-22 atomes de C ou des esters partiaux de polyols ayant 3-6 atomes de C avec des acides gras saturés ayant 14-22 atomes de C et

    D) 1-6 parties en poids d'eau à une température au dessus ou à l'intérieur de la zone de température d'inversion des phases, en une émulsion d'huile dans l'eau faiblement visqueuse, très finement divisée,

    caractérisé en ce qu'on épaissit ensuite celle-ci par introduction d'un donneur de consistance lipophile du type des co-émulsionnants indiqués dans C) et/ou d'un polymère soluble dans l'eau, jusqu'à la consistance d'une crème, dans laquelle l'émulsion présente à 20°C un comportement plastique avec une limite d'écoulement d'au moins 5 pascals, de préférence de 10 à 50 pascals.

2. Procédé selon la revendication caractérisé en ce que l'épaississement a lieu par introduction dans l'émulsion de 0,01 - 0,3 parties en poids d'un donneur de consistance lipophile ou par addition d'une solution aqueuse de 0,005-0,1 parties en poids d'un polymère soluble dans l'eau avec un poids moléculaire moyen de 500.000-5.000.000 de daltons.

3. Procédé selon les revendications 1 ou 2, caractérisé en ce que comme émulsionnant non ionique (B) on utilise un produit d'addition de 8-16 moles d'oxyde d'éthylène sur un alcool gras saturé ayant 16-22 atomes de C.

4. Procédé selon une des revendications 1-3, caractérisé en ce que le composant de l'huile (1) se compose de :

    50-100 % en poids d'une huile d'hydrocarbure et de

    0-25 % en poids d'un mono et/ou diester des formules

    $R^1$-COO-$R^2$
    $R^2$-OOC-$R^3$-COO-$R^2$

    et

    $R^1$-COO-$R^3$-OOC-$R^1$ ,

    dans lesquelles $R^1$ et $R^2$ sont des groupes alkyle avec 1-22 atomes de C ou des groupes alcényle avec 8-22 atomes de C et $R^3$ des groupes alkyléne avec 2-16 atomes de C et qui contiennent au moins 10 atomes de C, et de

    0-25 % en poids d'un triglycéride d'acides gras ayant 8-22 atomes de C.

5. Procédé selon une des revendiations 1-4, caractérisé en ce que le donneur de consistance lipophile est sélectionné dans le groupe de l'alcool cétylique, de l'alcool stéarylique, du monostéarate de glycérol et de mélanges de mono et distéarate de glycérol.

6. Procédé selon une des revendications 1-5, caractérisé en ce que le polymère soluble dans l'eau est sélectionné à partir de polymères d' acide acrylique réticulés ou de copolymères ayant un poids moléculaire moyen de 500.000 à 5.000.000 sous la forme des sels solubles dans l'eau et' d'éthers de cellulose non ioniques, solubles dans l'eau ou de leurs mélanges.